# EUROPÄISCHE PATENTANMELDUNG

(11) **EP 3 726 537 A1**
(43) Veröffentlichungstag der Anmeldung: **21.10.2020**
(21) Anmeldenummer: 19169814.1
(22) Anmeldetag: 17.04.2019
(51) Int. Cl.: G21K 1/10, A61B 6/00

(54) **ADAPTIVES RÖNTGENFILTER**

(71) Anmelder: Siemens Healthcare GmbH, 91052 Erlangen (DE)
(72) Erfinder: Rührig, Manfred, 91207 Lauf a.d. Pegnitz (DE)

(57) **Zusammenfassung**

Die Erfindung betrifft ein adaptives Röntgenfilter (10) für ein Festlegen einer Intensität einer Röntgenstrahlung, welches
- ein Röntgenfiltersystem (11) und
- ein Pumpensystem (12) aufweist,
- wobei das Röntgenfiltersystem (11)
- eine Röntgenstrahlung absorbierende Flüssigkeit aufweisende Röntgenfilterkammer (13) und
- ein bewegbares Stellelement (14) aufweist, wobei das bewegbare Stellelement zu einem Festlegen eines Volumens der Röntgenfilterkammer ausgebildet ist und wobei die Intensität der Röntgenstrahlung von dem Volumen der Röntgenfilterkammer abhängt,
- wobei das Pumpensystem
- eine volumenkonstante Kavität (15),
- ein erstes volumenvariables Verdrängungselement (16) und
- ein zweites volumenvariables Verdrängungselement (17) aufweist, welches als Gegenstück zum ersten volumenvariablen Verdrängungselement (16) ausgebildet ist und mittels der volumenkonstanten Kavität (15) mit dem ersten volumenvariablen Verdrängungselement (16) verbunden ist sowie ein geschlossenes System (18) bildet,
- wobei das zweite volumenvariable Verdrängungselement (17) mit dem bewegbaren Stellelement (14) derart mechanisch gekoppelt ist, dass eine Auslenkung des zweiten volumenvariablen Verdrängungselements (17) mit einer Auslenkung des bewegbaren Stellelements (14) korreliert, und
- wobei das erste volumenvariable Verdrängungselement (16) außerhalb der Röntgenstrahlung angeordnet ist.

## Beschreibung

Die Erfindung betrifft ein adaptives Röntgenfilter für ein Festlegen einer Intensität einer Röntgenstrahlung.

Ein adaptives Röntgenfilter wird üblicherweise dazu verwendet, eine Intensität einer Röntgenstrahlung gemäß einem mit der Röntgenstrahlung zu untersuchenden Patienten ortsabhängig festzulegen. Typischerweise weist der Patient ein unterschiedliches Absorptionsverhalten je nach zu untersuchendem Bereich des Patienten auf. Das Absorptionsverhalten unterscheidet sich insbesondere von Organ zu Knochen, weshalb die Intensität der Röntgenstrahlung vorzugsweise derart festgelegt wird, dass ein medizinisches Bild des Organs und des Knochens gleichmäßig ausgeleuchtet und damit diagnostisch aussagekräftig sein kann. Ein weiterer Grund für das Festlegen der Intensität der Röntgenstrahlung mittels des adaptiven Röntgenfilters ist üblicherweise, dass eine Dosis der bildgebenden Messung, welche mit der Intensität der Röntgenstrahlung korreliert, für den Patienten nicht höher als nötig ist und dennoch die diagnostische Aussagekraft gewährleistet ist.

Aus der DE 10 2012 206 953 B3 ist ein adaptives Röntgenfilter zur Veränderung der lokalen Intensität einer Röntgenstrahlung mit einer Röntgenstrahlung absorbierenden ersten Flüssigkeit und hydraulisch bewegten Stellelementen, die die Schichtdicke der ersten Flüssigkeit am Ort des jeweiligen Stellelements verändern, bekannt.

Eine Möglichkeit derartige hydraulisch bewegte Stellelemente einzustellen ist ein Abfahren einer Druckrampe einer Pumpe, wobei Ansteuerventile der jeweiligen Stellelemente kurzzeitig öffnen, wenn die gemäß dem Patienten vorgegebene Intensität der Röntgenstrahlung erreicht wird. Üblicherweise ist allerdings das Abfahren der Druckrampe langsam und/oder erfordert eine präzise Kontrolle der Pumpe. Weiterhin muss für ein Anpassen des jeweiligen Stellelements die Druckrampe erneut abgefahren oder das jeweilige Stellelement muss präzise entlüftet werden. Außerdem ist ein Aufbau der Stellelemente mit den Ansteuerventilen beliebig komplex.

Weitere Möglichkeiten zum Einstellen von Stellelementen stellt ein Einsatz von einem Elektromagneten und/oder einer Piezokeramik dar, wobei diese Steuerelemente üblicherweise aus relativ stark absorbierenden Materialen, insbesondere Kupferspulen und/oder ferromagnetischen Bauteilen, besteht.

Der Erfindung liegt die Aufgabe zu Grunde, ein adaptives Röntgenfilter für ein präzises und schnelles Festlegen einer Intensität einer Röntgenstrahlung anzugeben.

Die Aufgabe wird durch die Merkmale der unabhängigen Ansprüche gelöst. Vorteilhafte Ausgestaltungen sind in den Unteransprüchen beschrieben.

Das erfindungsgemäße adaptive Röntgenfilter für ein Festlegen einer Intensität einer Röntgenstrahlung weist
- ein Röntgenfiltersystem und
- ein Pumpensystem auf,
- wobei das Röntgenfiltersystem
- eine Röntgenstrahlung absorbierende Flüssigkeit aufweisende Röntgenfilterkammer und
- ein bewegbares Stellelement aufweist, wobei das bewegbare Stellelement zu einem Festlegen eines Volumens der Röntgenfilterkammer ausgebildet ist und wobei die Intensität der Röntgenstrahlung von dem Volumen der Röntgenfilterkammer abhängt,
- wobei das Pumpensystem
- eine volumenkonstante Kavität,
- ein erstes volumenvariables Verdrängungselement und
- ein zweites volumenvariables Verdrängungselement aufweist, welches als Gegenstück zum ersten volumenvariablen Verdrängungselement ausgebildet ist und mittels der volumenkonstanten Kavität mit dem ersten volumenvariablen Verdrängungselement verbunden ist sowie ein geschlossenes System bildet,
- wobei das zweite volumenvariable Verdrängungselement mit dem bewegbaren Stellelement derart mechanisch gekoppelt ist, dass eine Auslenkung des zweiten volumenvariablen Verdrängungselements mit einer Auslenkung des bewegbaren Stellelements korreliert, und
- wobei das erste volumenvariable Verdrängungselement außerhalb der Röntgenstrahlung angeordnet ist.

Das adaptive Röntgenfilter bietet insbesondere folgende Vorteile:
Das bewegbare Stellelement und damit das adaptive Röntgenfilter ist vorzugsweise im Wesentlichen röntgentransparent, insbesondere wenn das Volumen der Röntgenfilterkammer minimal ist. Die Röntgentransparenz des adaptiven Röntgenfilters wird beispielsweise dadurch positiv beeinflusst, dass das erste volumenvariable Verdrängungselement außerhalb der Röntgenstrahlung angeordnet ist. In anderen Worten wird typischerweise lediglich das bewegbare Stellelement und nicht das erste volumenvariable Verdrängungselement mit der Röntgenstrahlung durchleuchtet. Ein weiterer Vorteil kann sein, dass das erste volumenvariable Verdrängungselement mit einem elektromagnetisch, piezoelektrisch oder elektrostatisch betriebenen Aktor eingestellt werden kann, wobei der Aktor vorzugsweise außerhalb der Röntgenstrahlung angeordnet ist.

Das geschlossene Pumpensystem ist insbesondere daher vorteilhaft, weil dadurch eine variable Auslenkung des bewegbaren Stellelements schnell und/oder präzise ermöglicht wird.

Das adaptive Röntgenfilter ist typischerweise zwischen einer die Röntgenstrahlung emittierenden Röntgenröhre und einem bei einer bildgebenden Messung zu untersuchenden Patienten angeordnet. In diesem Fall kann die Intensität der Röntgenstrahlung vor einer Wechselwirkung in dem Patienten mittels des adaptiven Röntgenfilters festgelegt werden. Das Festlegen der Intensität der Röntgenstrahlung umfasst insbesondere ein Anpassen, vorzugsweise ein Erhöhen, besonders vorteilhafterweise ein Verringern der Intensität der Röntgenstrahlung. Das Festlegen der Intensität der Röntgenstrahlung umfasst insbesondere ein Festlegen einer im adaptiven Röntgenfilter erfolgenden Absorption der Röntgenstrahlung. Die Intensität der Röntgenstrahlung kann insbesondere einer Röntgenstrahlendosis im Patienten entsprechen. Die Röntgenstrahlung weist insbesondere elektromagnetische Wellen mit einer Photonenenergie zwischen ungefähr 100 eV und 250 keV, vorzugsweise zwischen 20 keV und 150 keV, besonders vorteilhafterweise zwischen 80 keV und 120 keV auf.

Das Röntgenfiltersystem ist insbesondere zu einem Filtern und/oder zu einem Absorbieren der Röntgenstrahlung ausgebildet. Das Röntgenfiltersystem ist insbesondere zu einem Festlegen der Intensität der Röntgenstrahlung in Abhängigkeit von einem Volumen an der Röntgenstrahlung absorbierenden Flüssigkeit in der Röntgenfilterkammer ausgebildet. In anderen Worten absorbiert das adaptive Röntgenfilter desto mehr Röntgenstrahlung, je größer das Volumen der Röntgenfilterkammer ist. Typischerweise korreliert, vorzugsweise korrespondiert, das Volumen der Röntgenfilterkammer mit dem Volumen der Röntgenstrahlung absorbierenden Flüssigkeit. Die Röntgenstrahlung absorbierende Flüssigkeit kann beispielsweise Galistan sein. Das Röntgenfiltersystem ist beispielsweise in der DE 10 2012 206 953 B3 offenbart.

Das Pumpensystem ist vorzugsweise zu einem Steuern, insbesondere zu einem Bewegen des bewegbaren Stellelements des Röntgenfiltersystems ausgebildet. Das Pumpensystem, insbesondere das zweite volumenvariable Verdrängungselement, ist vorzugsweise röntgentransparent.

Die volumenkonstante Kavität kann insbesondere ein Schlauch und/oder eine Röhre sein. Die volumenkonstante Kavität ist insbesondere derart ausgebildet, dass das Volumen der Kavität unveränderlich ist, vorzugsweise unabhängig eines Innendrucks des geschlossenen Systems. Ein Innendruck der Röntgenfilterkammer wirkt typischerweise dem Innendruck des geschlossenen Systems entgegen oder umgekehrt. Typischerweise ist der Innendruck der Röntgenfilterkammer und/oder der Innendruck des geschlossenen System derart anpassbar, dass eine Differenz zwischen dem Innendruck der Röntgenfilterkammer und dem Innendruck des geschlossenen Systems in einem Fall positiv, in einem weiteren Fall negativ oder in einem anderen Fall null ist. Insbesondere das Anpassen des Innendrucks der Röntgenfilterkammer und/oder des Innendrucks des geschlossenen Systems ermöglicht das Festlegen der Auslenkung des ersten volumenvariablen Verdrängungselements und/oder des zweiten volumenvariablen Verdrängungselements.

Zwischen dem ersten volumenvariablen Verdrängungselement und dem zweiten volumenvariablen Verdrängungselement ist typischerweise die volumenkonstante Kavität angeordnet. Die volumenkonstante Kavität verbindet vorzugsweise derart das erste volumenvariable Verdrängungselement mit dem zweiten volumenvariablen Verdrängungselement, dass das geschlossene System mit einem konstanten Gesamtvolumen gebildet wird. Das geschlossene System kann ein Arbeitsmedium, insbesondere ein Gas und/oder Öl und/oder Wasser bzw. eine Mischung der vorstehenden Elemente, aufweisen. Das Arbeitsmedium ist vorzugsweise röntgentransparent. Das geschlossene System ist vorzugsweise hermetisch dicht und/oder druckdicht ausgebildet.

Das geschlossene System ermöglicht insbesondere ein instantanes und/oder sofortiges Verschieben eines Deltavolumens innerhalb des ersten volumenvariablen Verdrängungselements, der volumenkonstanten Kavität und des zweiten volumenvariablen Verdrängungselements. Eine Größe des Deltavolumens ist üblicherweise variabel und/oder kontinuierlich festlegbar. Das Deltavolumen ist üblicherweise gleich oder kleiner als das Volumen des ersten volumenvariablen Verdrängungselements und/oder des zweiten volumenvariablen Verdrängungselements.

Wenn beispielsweise das erste volumenvariable Verdrängungselement um das Deltavolumen verändert wird, wird vorzugsweise das zweite volumenvariable Verdrängungselement um das Deltavolumen kompensierend verändert, wobei insbesondere das Gesamtvolumen konstant bleibt. In anderen Worten: wenn beispielsweise das Volumen des ersten volumenvariablen Verdrängungselements kleiner wird, wird vorzugsweise das Volumen des zweiten volumenvariablen Verdrängungselements, insbesondere im selben Maß, größer. Besonders vorteilhaferweise bleibt das Volumen der volumenkonstanten Kavität unverändert, während sich das Volumen des ersten volumenvariablen Verdrängungselements und das Volumen des zweiten volumenvariablen Verdrängungselements verändern. Das erste volumenvariable Verdrängungselement sowie das zweite volumenvariable Verdrängungselement sind vorzugsweise zueinander, insbesondere in Hinblick auf das jeweilige Volumen, vergleichbar ausgebildet.

Die Auslenkung des zweiten volumenvariablen Verdrängungselement korreliert insbesondere mit einer Veränderung des zweiten volumenvariablen Verdrängungselement gemäß dem Deltavolumen. Die Auslenkung beschreibt insbesondere ein Anpassen eines Abstands zwischen einem beweglichen Teil des zweiten volumenvariablen Verdrängungselements und der volumenkonstanten Kavität. Beim Auslenken vergrößert oder verkleinert oder verschiebt sich insbesondere das zweite volumenvariable Verdrängungselement, vorzugsweise der bewegliche Teil des zweiten volumenvariablen Verdrängungselements. Das zweite volumenvariable Verdrängungselement und damit das bewegbare Stellelement kann vorzugsweise in Abhängigkeit vom ersten volumenvariablen Verdrängungselement ausgelenkt werden. Das Auslenken kann ein Herauslenken und/oder ein Zurückstellen umfassen.

Die Auslenkung des bewegbaren Stellelements beeinflusst insbesondere unmittelbar das Volumen der Röntgenfilterkammer. Wenn das zweite volumenvariable Verdrängungselement ausgelenkt wird, wird insbesondere das Volumen der Röntgenfilterkammer vorzugsweise instantan entsprechend angepasst. Die Auslenkung des zweiten volumenvariablen Verdrängungselements korreliert insbesondere derart mit der Auslenkung des bewegbaren Stellelements, dass das bewegbare Stellelement verschoben wird, wenn das zweite volumenvariable Verdrängungselement, insbesondere der bewegliche Teil des zweiten volumenvariablen Verdrängungselements seine Position verändert.

Die mechanische Kopplung zwischen dem zweiten volumenvariablen Verdrängungselement mit dem bewegbaren Stellelement ist vorzugsweise starr und/oder fest. Die mechanische Kopplung kann mittels einer Koppelstange und/oder einem Gelenk erfolgen.

Bei der bildgebenden Messung wird das erste volumenvariable Verdrängungselement vorzugsweise nicht durchleuchtet. Das zweite volumenvariable Verdrängungselement ist üblicherweise innerhalb der Röntgenstrahlung angeordnet, typischerweise aufgrund der mechanischen Kopplung mit dem bewegbaren Stellelement. Die volumenkonstante Kavität kann zumindest teilweise außerhalb der Röntgenstrahlung angeordnet sein.

Das Festlegen der Intensität der Röntgenstrahlung erfolgt üblicherweise derart, dass das Volumen des ersten volumenvariablen Verdrängungselements um das Deltavolumen verkleinert wird, wodurch sich das Volumen des zweiten volumenvariablen Verdrängungselement um das Deltavolumen vergrößert, und dass aufgrund der Vergrößerung des Volumens des zweiten volumenvariablen Verdrängungselements das mechanisch gekoppelte bewegbare Stellelement derart auslenkt, um gemäß dem damit festgelegten Volumen der Röntgenfilterkammer die Intensität der Röntgenstrahlung festzulegen. Das Festlegen der Intensität der Röntgenstrahlung kann mehrmals nacheinander und/oder stufenlos, insbesondere während der bildgebenden Messung, erfolgen. Das Festlegen der Intensität der Röntgenstrahlung kann ein Erhöhen oder ein Verringern der Intensität der Röntgenstrahlung insbesondere relativ zu einem vorherigen Festlegen der Intensität der Röntgenstrahlung umfassen.

Eine Ausführungsform sieht vor, dass ein Übersetzungsverhältnis zwischen einer Auslenkung des ersten volumenvariablen Verdrängungselements und der Auslenkung des zweiten volumenvariablen Verdrängungselements größer oder kleiner als 1 ist. Die Auslenkung des ersten volumenvariablen Verdrängungselements beschreibt insbesondere ein Anpassen eines Abstands zwischen einem beweglichen Teil des ersten volumenvariablen Verdrängungselements und der volumenkonstanten Kavität. Je nach Ausführung des ersten volumenvariablen Verdrängungselements und des zweiten volumenvariablen Verdrängungselements variiert zwar insbesondere die jeweilige Auslenkung, aber das Deltavolumen wird dennoch gleichermaßen kompensiert. Diese Ausführungsform ist besonders vorteilhaft, weil je nach Betrag des Übersetzungsverhältnisses eine Über- bzw. Untersetzung zwischen dem ersten volumenvariablen Verdrängungselement und dem zweiten volumenvariablen Verdrängungselement, insbesondere ein Hebel zwischen dem ersten volumenvariablen Verdrängungselement und dem zweiten volumenvariablen Verdrängungselement besteht. Beispielsweise kann das Übersetzungsverhältnis derart festgelegt sein, dass das bewegbare Stellelement und somit das Volumen der Röntgenfilterkammer sich mit einer vergleichsweise hohen Präzision oder einer vergleichsweise großen Geschwindigkeit einstellen lässt.

Eine Ausführungsform sieht vor, dass das zweite volumenvariable Verdrängungselement eine Membran oder ein Kolben ist. Insbesondere der bewegliche Teil des zweiten volumenvariablen Verdrängungselements kann die Membran oder der Kolben sein. Diese Ausführungsform ist insbesondere vorteilhaft, weil diese einfach zu realisieren ist. Die Membran bietet eine im Gewicht leichte und/oder typischerweise röntgentransparente Realisierung. Der Kolben ermöglicht typischerweise eine starrere und/oder festere mechanische Kopplung mit dem bewegbaren Steuerelement. Der Kolben ist üblicherweise für einen höheren Innendruck des geschlossenen Systems im Vergleich zur Membran geeignet. Das erste volumenvariable Verdrängungselement kann insbesondere unabhängig von dem zweiten volumenvariablen Verdrängungselement eine Membran oder ein Kolben sein. Die Membran ist typischerweise elastisch, insbesondere derart, dass das Deltavolumen aufgenommen oder abgegeben werden kann. Die Membran kann ein Textil oder Plastikmaterial aufweisen.

Grundsätzlich ist insbesondere ein Aktor zum Einstellen der Auslenkung des ersten volumenvariablen Verdrängungselement ausgebildet. Der Aktor kann insbesondere einen Motor, einen Hebel und/oder einen Schalter aufweisen. Aufgrund des geschlossenen Systems kann vorzugsweise mittels des Aktors das Volumen der Röntgenfilterkammer festgelegt werden. Der Aktor wirkt insbesondere dem Innendruck der Röntgenfilterkammer entgegen. Der Aktor kann vorzugsweise die Auslenkung des ersten volumenvariablen Verdrängungselements mehrmals, insbesondere nacheinander, während der bildgebenden Messung einstellen.

Eine Ausführungsform sieht vor, dass das erste volumenvariable Verdrängungselement ein Kolben ist, wobei das Pumpensystem einen Aktor aufweist und wobei der Aktor gemeinsam mit dem Kolben des ersten volumenvariablen Verdrängungselements eine Kolbenpumpe bildet. Die Kolbenpumpe ist insbesondere aufgrund eines vergleichsweise hohen möglichen Innendrucks des geschlossenen Systems vorteilhaft.

Eine Ausführungsform sieht vor, dass das erste volumenvariable Verdrängungselement eine Membran ist. Die Membran ermöglicht insbesondere eine im Gewicht leichte und/oder röntgentransparente Realisierung des ersten volumenvariablen Verdrängungselements. Alternativ kann das erste volumenvariable Verdrängungselement eine Membran und ein Reservoir aufweisen. Insbesondere die Membran ist für eine Auslenkung und/oder eine Verdrängung ausgebildet.

Eine Ausführungsform sieht vor, dass das Pumpensystem den Aktor aufweist, wobei der Aktor eine elektromagnetische Spule und einen Magneten aufweist, wobei die elektromagnetische Spule oder der Magnet auf der Membran des ersten volumenvariablen Verdrängungselements angeordnet ist und wobei eine Auslenkung des ersten volumenvariablen Verdrängungselements mit einem Abstand zwischen der elektromagnetischen Spule und dem Magneten korreliert. Der Magnet kann beispielsweise ein Dauermagnet sein. Der Abstand zwischen der elektromagnetischen Spule und dem Magneten kann insbesondere gemäß einem in der elektromagnetischen Spule fließenden Strom festgelegt sein. Diese Ausführungsform ist insbesondere vorteilhaft, weil ein derartiger elektromagnetischer Aktor vorzugsweise quasi verschleißfrei und/oder insbesondere kompakt aufgebaut sein kann.

Eine Ausführungsform sieht vor, dass die elektromagnetische Spule als Leiterbahn ausgebildet ist. Diese Ausführungsform ist insbesondere in Kombination mit dem elektromagnetischen Aktor vorteilhaft und ermöglicht insbesondere ein besonders kompaktes adaptives Röntgenfilter. Die Leiterbahn kann vorzugsweise lithographisch und/oder als Teil einer Platine hergestellt worden sein. Diese Ausführungsform bietet eine insbesondere kompakte Realisierung.

Eine Ausführungsform sieht vor, dass das Pumpensystem einen Aktor aufweist und dass der Aktor gemeinsam mit dem ersten volumenvariablen Verdrängungselement einen piezoelektrischen oder elektrostatischen Aktor bildet. Diese Ausführungsform ist insbesondere eine Alternative zu der Ausführung des ersten volumenvariablen Verdrängungselements als Kolbenpumpe oder elektromagnetischem Aktor und ermöglicht vorteilhafterweise ein schnelles Festlegen der Intensität der Röntgenstrahlung.

Eine Ausführungsform sieht vor, dass das Röntgenfiltersystem mehrere Röntgenfilterkammern und mehrere bewegbare Stellelemente aufweist und dass die mehreren Röntgenfilterkammern und die mehreren bewegbaren Stellelemente in einer Ebene senkrecht zur Röntgenstrahlung angeordnet sind. Die mehreren Röntgenfilterkammern und die mehreren bewegbaren Stellelemente sind vorteilhafterweise matrixförmig angeordnet. Besonders vorteilhafterweise wirkt zumindest ein bewegbares Stellelement der mehreren bewegbaren Stellelement als Röntgenfilter für einen vorgegebenen oder mehrere vorgegebene Pixel eines Röntgendetektors, mit welchem die Röntgenstrahlung bei der bildgebenden Messung erfasst wird. Dieses Ausführungsbeispiel ermöglicht insbesondere ein Festlegen der Intensität der Röntgenstrahlung in einer, besonders vorteilhafterweise in zwei Dimensionen. Die mehreren bewegbaren Stellelemente und die mehreren Röntgenfilterkammern sind insbesondere parallelgeschaltet und/oder vorzugsweise separat einzeln oder in Gruppen miteinander verbunden, damit das adaptive Filter eine Intensitätsverteilung der Röntgenstrahlung ermöglicht. Die Intensitätsverteilung kann einer Graustufenverteilung entsprechen. Diese Ausführungsform ist insbesondere vorteilhaft für die medizinische Bildgebung, weil die Intensität der Röntgenstrahlung ortsabhängig festgelegt wird.

Eine Ausführungsform sieht vor, dass das geschlossene System des Pumpensystems ein Gesamtvolumen kleiner als 10 mL, vorzugsweise kleiner als 1 mL, besonders vorteilhafterweise kleiner als 100 µL aufweist. Diese Ausführungsform ermöglicht insbesondere ein vergleichsweise schnelleres Festlegen der Intensität der Röntgenstrahlung, weil das Deltavolumen relativ zum Gesamtvolumens vergleichsweise gering ist.

Die Röntgeneinrichtung weist
- eine Röntgenröhre für ein Emittieren der Röntgenstrahlung,
- das adaptive Röntgenfilter und
- ein Steuersystem auf, welches gemäß einem bei einer bildgebenden Messung zu untersuchenden Patienten das Pumpensystem steuert. Das Steuern des Pumpensystems umfasst insbesondere das Festlegen der Intensität der Röntgenstrahlung.

Die Röntgeneinrichtung kann insbesondere einen C-Bogen umfassen. Alternativ kann die Röntgeneinrichtung einen Computertomographen umfassen. Die Röntgeneinrichtung ist insbesondere zu einem Durchführen der bildgebenden Messung ausgebildet. Die bildgebende Messung kann eine kontrastmittelgestützte Angiographie und/oder eine Durchleuchtung und/oder eine zeitaufgelöste Messung umfassen. Die bildgebende Messung kann ein Organ und/oder eine anatomische Struktur des Patienten betreffen.

Im Folgenden wird die Erfindung anhand der in den Figuren dargestellten Ausführungsbeispiele näher beschrieben und erläutert. Grundsätzlich werden in der folgenden Figurenbeschreibung im Wesentlichen gleichbleibende Strukturen und Einheiten mit demselben Bezugszeichen wie beim erstmaligen Auftreten der jeweiligen Struktur oder Einheit benannt.

Es zeigen:
Fig. 1 ein adaptives Röntgenfilter in einem ersten Ausführungsbeispiel,
Fig. 2 ein adaptives Röntgenfilter in einem zweiten Ausführungsbeispiel,
Fig. 3 ein adaptives Röntgenfilter in einem dritten Ausführungsbeispiel,
Fig. 4 ein adaptives Röntgenfilter in einem vierten Ausführungsbeispiel und
Fig. 5 eine Röntgeneinrichtung in einem fünften Ausführungsbeispiel.

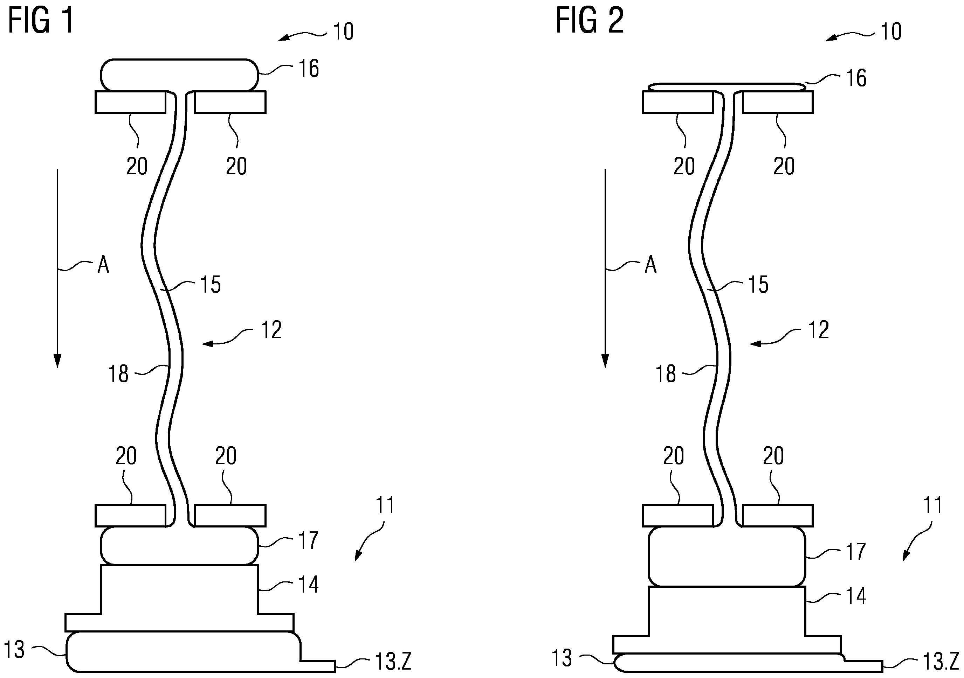

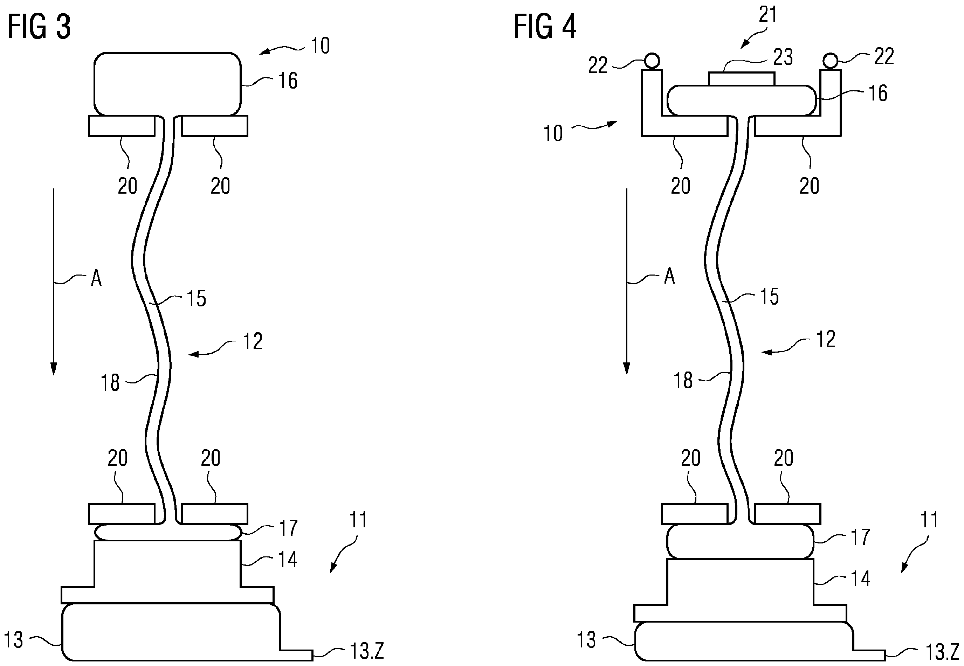

**Fig. 1** zeigt ein adaptives Röntgenfilter 10 für ein Festlegen einer Intensität einer Röntgenstrahlung in einem ersten Ausführungsbeispiel. Das erste Ausführungsbeispiel zeigt das adaptive Röntgenfilter 10 beispielsweise in einem Zwischenzustand bezüglich der Intensität der Röntgenstrahlung, während die in Fig. 2 und 3 gezeigten Ausführungsbeispiele mögliche Extrempositionen des adaptiven Röntgenfilter 10 zeigen. Ein Wert der Intensität der Röntgenstrahlung gemäß dem Zwischenzustand liegt typischerweise zwischen dem Wert der beiden Extrempositionen.

Das adaptive Röntgenfilter 10 weist ein Röntgenfiltersystem 11 und ein Pumpensystem 12 auf. Das Röntgenfiltersystem 11 weist eine Röntgenstrahlung absorbierende Flüssigkeit aufweisende Röntgenfilterkammer 13 und ein bewegbares Stellelement 14 auf. Das bewegbare Stellelement 14 ist als Stempel ausgebildet. Das bewegbare Stellelement 14 ist zu einem Festlegen eines Volumens der Röntgenfilterkammer 13 ausgebildet. Die Röntgenfilterkammer 13 ist über eine Zuleitung 13.Z mit einem nicht gezeigten Flüssigkeitsreservoir verbunden, in welches in Abhängigkeit von einer Position des bewegbaren Stellelements 14 relativ zur Röntgenfilterkammer 13 die Röntgenstrahlung absorbierende Flüssigkeit zurückfließt. Die Intensität der Röntgenstrahlung hängt von dem Volumen der Röntgenfilterkammer 13 ab. Die Röntgenfilterkammer 13 hat typischerweise ein Volumen kleiner als 10 mL. Die Röntgenfilterkammer 13 hat typischerweise ein Volumen zwischen 0,001 mL und 1 mL, besonders vorteilhafterweise zwischen 0,01 mL und 0,1 mL.

Das Pumpensystem 12 weist eine volumenkonstante Kavität 15, ein erstes volumenvariables Verdrängungselement 16 und ein zweites volumenvariables Verdrängungselement 17 auf. Das zweite volumenvariable Verdrängungselement 17 ist als Gegenstück zum ersten volumenvariablen Verdrängungselement 16 ausgebildet und ist mittels der volumenkonstanten Kavität 15 mit dem ersten volumenvariablen Verdrängungselement 17 verbunden. Mittels der volumenkonstanten Kavität 15 sowie mit dem ersten volumenvariablen Verdrängungselement 16 bildet das zweite volumenvariable Verdrängungselement 17 ein geschlossenes System 18. Vorzugsweise weist das geschlossene System des Pumpensystems 12 ein Gesamtvolumen kleiner als 10 mL auf. Das Volumen des ersten volumenvariablen Verdrängungselements 16 entspricht in diesem Ausführungsbeispiel dem Volumen des zweiten volumenvariablen Verdrängungselements 17.

Das zweite volumenvariable Verdrängungselement 17 ist in diesem Ausführungsbeispiel eine Membran, kann alternativ allerdings ein Kolben sein.

Das zweite volumenvariable Verdrängungselement 17 ist mit dem bewegbaren Stellelement 14 derart mechanisch gekoppelt, dass eine Auslenkung des zweiten volumenvariablen Verdrängungselements 17 mit einer Auslenkung des bewegbaren Stellelements 14 korreliert.

Das erste volumenvariable Verdrängungselement 16 ist außerhalb der Röntgenstrahlung angeordnet. Beispielsweise kann die nicht in Fig. 1 gezeigte Röntgenröhre 19 zwischen dem ersten volumenvariablen Verdrängungselement 16 und dem zweiten volumenvariablen Verdrängungselement 17 angeordnet sein. Alternativ oder zusätzlich kann das erste volumenvariable Verdrängungselement 16 in einer Ebene senkrecht zur Röntgenstrahlung versetzt angeordnet sein.

Die Befestigungselemente 20 dienen als Referenzpunkte und ermöglichen insbesondere ein Verschieben eines Deltavolumens in dem geschlossenen System 18 und/oder das Auslenken des bewegbaren Stellelements 14. Die Befestigungselemente 20 sind insbesondere relativ zum ersten volumenvariablen Verdrängungselement 16, zum zweiten volumenvariablen Verdrängungselement 17 und zum bewegbaren Stellelement 13 ortsfest.

Das Auslenken erfolgt insbesondere parallel zur Achse A. Ein beweglicher Teil des ersten volumenvariablen Verdrängungselements 16 und/oder ein beweglicher Teil des zweiten volumenvariablen Verdrängungselements 17 sind insbesondere entlang der Achse A auslenkbar. Der bewegliche Teil des ersten volumenvariablen Verdrängungselements 16 ist insbesondere ein den Befestigungselementen 20 gegenüberliegender Bereich. Der bewegliche Teil des zweiten volumenvariablen Verdrängungselements 17 ist insbesondere ein an dem bewegbaren Stellelement 14 anliegender Bereich.

In diesem Ausführungsbeispiel ist ein Übersetzungsverhältnis zwischen einer Auslenkung des ersten volumenvariablen Verdrängungselements 16 und der Auslenkung des zweiten volumenvariablen Verdrängungselements 17 gleich 1. Grundsätzlich ist es allerdings denkbar, dass das Übersetzungsverhältnis größer oder kleiner als 1 ist. Das Übersetzungsverhältnis hängt insbesondere von einer auslenkenden Fläche des ersten volumenvariablen Verdrängungselements 16 und des zweiten volumenvariablen Verdrängungselements 17 ab.

**Fig. 2** zeigt das adaptive Röntgenfilter 10 in einem zweiten Ausführungsbeispiel. Im Vergleich zum ersten Ausführungsbeispiel ist das Volumen der Röntgenfilterkammer 13 aufgrund der Auslenkung des bewegbaren Stellelements 14 verkleinert. Das Volumen des ersten volumenvariablen Verdrängungselements 16 ist in diesem Ausführungsbeispiel kleiner als das Volumen des zweiten volumenvariablen Verdrängungselements 17. In diesem Fall ist im Vergleich zum ersten Ausführungsbeispiel ein Deltavolumen, welches im Wesentlichen dem Volumen des ersten volumenvariablen Verdrängungselements 16 entspricht, in Richtung des zweiten volumenvariablen Verdrängungselements 17 verschoben, wodurch das Volumen der Röntgenfilterkammer 13 verringert ist und damit die Intensität der Röntgenstrahlung erhöht wird. Das zweite Ausführungsbeispiel kann einer Extremposition des adaptiven Röntgenfilters 10 mit maximaler Intensität der Röntgenstrahlung entsprechen.

**Fig. 3** zeigt das adaptive Röntgenfilter 10 in einem dritten Ausführungsbeispiel. Das Volumen des ersten volumenvariablen Verdrängungselements 16 ist in diesem Ausführungsbeispiel größer als das Volumen des zweiten volumenvariablen Verdrängungselements 17. Im Vergleich zu den beiden vorherigen Ausführungsbeispielen ist das Volumen des zweiten volumenvariablen Verdrängungselement 17 minimal, wodurch das bewegbare Stellelement 14 in Richtung des ersten volumenvariablen Verdrängungselement 16 ausgelenkt wird, insbesondere aufgrund des Innendrucks in der Röntgenfilterkammer mit angeschlossenen Flüssigkeitsreservoir. Das dritte Ausführungsbeispiel kann einer Extremposition des adaptiven Röntgenfilters 10 mit minimaler Intensität der Röntgenstrahlung entsprechen.

**Fig. 4** zeigt das adaptive Röntgenfilter 10 in einem vierten Ausführungsbeispiel und entspricht im Wesentlichen dem ersten Ausführungsbeispiel insbesondere mit einem elektromagnetischen Aktor. Das erste volumenvariable Verdrängungselement 16 ist eine Membran. Das Pumpensystem 12 weist einen Aktor 21 auf, wobei der Aktor 21 eine elektromagnetische Spule 22 und einen Magneten 23 aufweist. Die elektromagnetische Spule 22 kann als Leiterbahn ausgebildet sein. Die elektromagnetische Spule 22 ist in diesem Ausführungsbeispiel an den Befestigungselementen 20 befestigt und ringförmig dargestellt. Der Magnet 23 ist auf der Membran des ersten volumenvariablen Verdrängungselements 16 angeordnet. Die Auslenkung des ersten volumenvariablen Verdrängungselements 16 korreliert mit einem Abstand zwischen der elektromagnetischen Spule 22 und dem Magneten 23. In anderen Worten kann in Abhängigkeit der Stromrichtung und der Stromamplitude in der elektromagnetischen Spule 22 der Abstand zwischen der elektromagnetischen Spule 22 und dem Magneten 23 derart festgelegt werden, dass damit ebenfalls die Intensität der Röntgenstrahlung festgelegt wird.

Alternativ zum elektromagnetischen Aktor kann das erste volumenvariable Verdrängungselement 16 ein Kolben sein, wobei das Pumpensystem 12 einen Aktor aufweist und wobei der Aktor gemeinsam mit dem Kolben des ersten volumenvariablen Verdrängungselements 16 eine Kolbenpumpe bildet. Eine weitere alternative Ausführungsform ist, dass das Pumpensystem 12 einen Aktor aufweist und wobei der Aktor gemeinsam mit dem ersten volumenvariablen Verdrängungselement 16 einen piezoelektrischen oder elektrostatischen Aktor bildet.

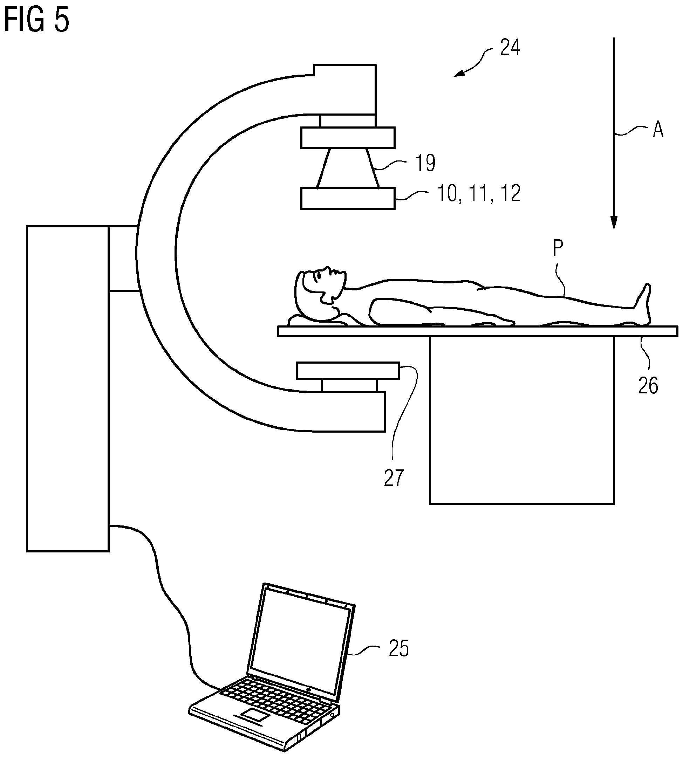

**Fig. 5** zeigt eine Röntgeneinrichtung 24 in einem fünften Ausführungsbeispiel. Die Röntgeneinrichtung 24 weist die Röntgenröhre 19 für das Emittieren der Röntgenstrahlung, das adaptive Röntgenfilter 10 und ein Steuersystem 25 auf. Das Steuersystem 25 steuert gemäß einem bei einer bildgebenden Messung zu untersuchenden Patienten P das Pumpensystem 12. Der Patient P ist auf einer Patientenliege 26 gelagert. Die Röntgeneinrichtung 25 weist einen Röntgendetektor 27 zum Erfassen der Röntgenstrahlung auf. Die Röntgenröhre 19, das adaptive Röntgenfilter 10 und der Röntgendetektor 27 sind an einem C-Bogen der Röntgeneinrichtung 24 befestigt. Das adaptive Röntgenfilter 10 ist zwischen der Röntgenröhre 19 und dem Patienten P angeordnet. Die Röntgenstrahlung ist parallel zur Achse A ausgerichtet. Das Röntgenfiltersystem 11 weist mehrere Röntgenfilterkammern und mehrere bewegbare Stellelemente auf, wobei die mehreren Röntgenfilterkammern und die mehreren bewegbaren Stellelemente in einer Ebene senkrecht zur Röntgenstrahlung angeordnet sind.

Das Steuersystem 25 ist zum Steuern der bildgebenden Messung mit der Röntgeneinrichtung 24 verbunden, wobei die Intensität der Röntgenstrahlung festgelegt wird. Das Steuersystem 25 kann ein Rekonstruktionssystem zu einem Bereitstellen eines medizinischen Bildes gemäß den am Röntgendetektor 27 erfassten Röntgenstrahlen erfassen.

Obwohl die Erfindung im Detail durch die bevorzugten Ausführungsbeispiele näher illustriert und beschrieben wurde, so ist die Erfindung dennoch nicht durch die offenbarten Beispiele eingeschränkt und andere Variationen können vom Fachmann hieraus abgeleitet werden, ohne den Schutzumfang der Erfindung zu verlassen.

## Patentansprüche

1. Adaptives Röntgenfilter (10) für ein Festlegen einer Intensität einer Röntgenstrahlung, aufweisend
- ein Röntgenfiltersystem (11) und
- ein Pumpensystem (12),
- wobei das Röntgenfiltersystem (11)
- eine Röntgenstrahlung absorbierende Flüssigkeit aufweisende Röntgenfilterkammer (13) und
- ein bewegbares Stellelement (14) aufweist, wobei das bewegbare Stellelement (14) zu einem Festlegen eines Volumens der Röntgenfilterkammer (13) ausgebildet ist und wobei die Intensität der Röntgenstrahlung von dem Volumen der Röntgenfilterkammer (13) abhängt,
**dadurch gekennzeichnet, dass** das Pumpensystem (12)
- eine volumenkonstante Kavität (15),
- ein erstes volumenvariables Verdrängungselement (16) und
- ein zweites volumenvariables Verdrängungselement (17) aufweist, welches als Gegenstück zum ersten volumenvariablen Verdrängungselement (16) ausgebildet ist und mittels der volumenkonstanten Kavität (15) mit dem ersten volumenvariablen Verdrängungselement (16) verbunden ist sowie ein geschlossenes System (18) bildet,
- wobei das zweite volumenvariable Verdrängungselement (17) mit dem bewegbaren Stellelement (14) derart mechanisch gekoppelt ist, dass eine Auslenkung des zweiten volumenvariablen Verdrängungselements (17) mit einer Auslenkung des bewegbaren Stellelements (14) korreliert, und
- wobei das erste volumenvariable Verdrängungselement (16) außerhalb der Röntgenstrahlung angeordnet ist.

2. Adaptives Röntgenfilter (10) nach Anspruch 1, wobei ein Übersetzungsverhältnis zwischen einer Auslenkung des ersten volumenvariablen Verdrängungselements (16) und der Auslenkung des zweiten volumenvariablen Verdrängungselements (17) größer oder kleiner als 1 ist.

3. Adaptives Röntgenfilter (10) nach einem der vorhergehenden Ansprüche, wobei das zweite volumenvariable Verdrängungselement (17) eine Membran oder ein Kolben ist.

4. Adaptives Röntgenfilter (10) nach einem der vorhergehenden Ansprüche, wobei das erste volumenvariable Verdrängungselement (16) ein Kolben ist, wobei das Pumpensystem (12) einen Aktor aufweist und wobei der Aktor gemeinsam mit dem Kolben des ersten volumenvariablen Verdrängungselements (16) eine Kolbenpumpe bildet.

5. Adaptives Röntgenfilter (10) nach einem der Ansprüche 1 bis 3, wobei das erste volumenvariable Verdrängungselement (16) eine Membran ist.

6. Adaptives Röntgenfilter (10) nach Anspruch 5, wobei das Pumpensystem (12) einen Aktor (21) aufweist, wobei der Aktor (21) eine elektromagnetische Spule (22) und einen Magneten (23) aufweist, wobei die elektromagnetische Spule (22) oder der Magnet (23) auf der Membran des ersten volumenvariablen Verdrängungselements (16) angeordnet ist und wobei eine Auslenkung des ersten volumenvariablen Verdrängungselements (16) mit einem Abstand zwischen der elektromagnetischen Spule (22) und dem Magneten (23) korreliert.

7. Adaptiver Röntgenfilter (10) nach Anspruch 6, wobei die elektromagnetische Spule (22) als Leiterbahn ausgebildet ist.

8. Adaptives Röntgenfilter (10) nach Anspruch 5, wobei das Pumpensystem (12) einen Aktor aufweist und wobei der Aktor gemeinsam mit dem ersten volumenvariablen Verdrängungselement (16) einen piezoelektrischen Aktor bildet.

9. Adaptives Röntgenfilter (10) nach Anspruch 5, wobei das Pumpensystem (12) einen Aktor aufweist und wobei der Aktor gemeinsam mit dem ersten volumenvariablen Verdrängungselement (16) einen elektrostatischen Aktor bildet.

10. Adaptives Röntgenfilter (10) nach einem der vorhergehenden Ansprüche, wobei das Röntgenfiltersystem (11) mehrere Röntgenfilterkammern und mehrere bewegbare Stellelemente aufweist und wobei die mehreren Röntgenfilterkammern und die mehreren bewegbaren Stellelemente in einer Ebene senkrecht zur Röntgenstrahlung angeordnet sind.

11. Adaptives Röntgenfilter (10) nach einem der vorhergehenden Ansprüche, wobei das geschlossene System (18) des Pumpensystems (12) ein Gesamtvolumen kleiner als 10 mL aufweist.

12. Röntgeneinrichtung (24) nach einem der vorhergehenden Ansprüche, aufweisend
- eine Röntgenröhre (19) für ein Emittieren der Röntgenstrahlung,
- das adaptive Röntgenfilter (10) nach einem der vorhergehenden Ansprüche und
- ein Steuersystem (25), welches gemäß einem bei einer bildgebenden Messung zu untersuchenden Patienten (P) das Pumpensystem (12) steuert.
